Europäisches Patentamt

European Patent Office

Office européen des brevets

(19)

(11) Veröffentlichungsnummer: **0 265 545 B1**

# EUROPÄISCHE PATENTSCHRIFT

(12)

(45) Veröffentlichungstag der Patentschrift: **06.03.91**

(51) Int. Cl.⁵: **A61M 15/00**

(21) Anmeldenummer: **86115030.8**

(22) Anmeldetag: **29.10.86**

(54) Inhalationsgerät.

(43) Veröffentlichungstag der Anmeldung:
**04.05.88 Patentblatt 88/18**

(45) Bekanntmachung des Hinweises auf die
Patenterteilung:
**06.03.91 Patentblatt 91/10**

(84) Benannte Vertragsstaaten:
**AT BE CH DE ES FR GB GR IT LI LU NL SE**

(56) Entgegenhaltungen:
**DE-A- 3 335 745**
**FR-A- 2 264 968**
**FR-A- 2 280 235**
**FR-A- 2 440 742**
**US-A- 4 243 893**

**PATENT ABSTRACTS OF JAPAN, Band 10,
Nr. 63 (C-332)[2120], 13. März 1986; & JP - A -
60 200 804 (TEIJIN) 11.10.1985**

(73) Patentinhaber: **Brugger, Stephan,
Dipl.-Wirt.-Ing.
Etztalstrasse 21
W-8137 Berg(DE)**

(72) Erfinder: **Der Erfinder hat auf seine Nennung
verzichtet**

(74) Vertreter: **Hoffmann, Klaus, Dr. rer. nat. et al
Hoffmann . Eitle & Partner Patentanwälte
Postfach 81 04 20 Arabellastrasse 4
W-8000 München 81(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die Erfindung betrifft ein Inhalationsgerät, mit einem Drucklufterzeuger, einem Elektromotor, der den Drucklufterzeuger antreibt, und einem im wesentlichen quaderförmigen Gehäuse, das im Bereich seiner Rückseite Lufteinlaßöffnungen und im Bereich seiner dem Benutzer zugewandten Frontseite Luftaustrittsöffnungen aufweist, so daß Kühlluft im uesentlichen horizontal von der Rückseite zur Frontseite hin durch das Innere des Gehäuses strömt.

Derartige Inhalationsgeräte dienen der therapeutischen Behandlung von Erkrankungen der Atemwege sowie der Lunge. Hierzu wird an den Druckluftanschluß des Geräts eine Zerstäubungsvorrichtung angeschlossen, die einen feinen Aerosol-Nebel erzeugt, der vom Patienten einzuatmen ist. Zusammen mit dem Aerosol inhaliert der Patient therapeutisch wirksame Substanzen, welche in den Flüssigkeitströpfchen gelöst sind.

Der im Inhalationsgerät enthaltene Drucklufterzeuger und der diesen antreibende Elektromotor werden im Betrieb warm; diese Abwärme muß aus dem Geräteinneren abgeführt werden. Zu diesem Zweck sind üblicherweise entsprechende Lüftungsschlitze im Gehäuse vorgesehen. Um zu verhindern, daß vom inhalierenden Patienten ausgeatmete, medikamentenhaltige Luft in das Innere des Geräts gelangt, wird die benötigte Kühlluft im Bereich der Rückseite des Gehäuses angesaugt und im Bereich der dem Benutzer zugewandten Frontseite wieder ausgeblasen. Auf ihrem Weg durch das Innere des Gerätegehäuses strei cht die Kühlluft am Drucklufterzeuger und dem Antriebsmotor vorbei, so daß deren Verlustwärme wirksam abgeführt wird.

Die Durchströmung des Gehäuseinneren von der Rückseite zur Vorderseite hin bringt allerdings den Nachteil mit sich, daß der meist unmittelbar vor dem Gerät sitzende Patient von der Kühlluft angeblasen wird. Dieser Luftzug wird oftmals als störend empfunden und kann sogar gesundheitsschädlich sein. In der Kühlluft enthaltene Schmutz- und Staubpartikel setzen sich bevorzugt in bzw. um die Luftaustrittsöffnungen ab. Die Folge ist eine starke Verschmutzungsneigung der dem Benutzer zugewandten Frontseite des Geräts. Gerade im medizinischen Bereich eingesetzte Geräte sollten aber aus hygienischen Gründen möglichst frei von Verschmutzung sein.

Die Erzeugung von Druckluft ist unvermeidbar mit einem gewissen Maß an Lärm verbunden. Die Geräuschemission eines druckluftbetriebenen Inhalationsgeräts kann deshalb nicht vernachlässigt werden. Einer Verringerung der Geräuschentwicklung während des Betriebs durch möglichst umfassende Schalldämpfung bzw. Abkapselung des Drucklufterzeugers steht die bereits angesprochene Notwendigkeit entgegen, beträchtliche Mengen von Kühlluft möglichst ungehindert durch das Geräteinnere zu leiten.

Angesichts dieser Probleme und Nachteile bei herkömmlichen, druckluferzeugenden Inhalationsgeräten ist es Aufgabe vorliegender Erfindung, ein derartiges Gerät so zu verbessern, daß bei hochwirksamer Kühlung des Drucklufterzeugers und des zugehörigen Elektromotors sowie unter grundsätzlicher Beibehaltung der Durchströmungsrichtung des Gehäuseinneren von hinten nach vorne die Geräuschemission auf ein Minimum reduziert wird, der Patient keine Störung durch ihm entgegengeblasene Kühlluft erleidet und die Gerätevorderseite möglichst frei von Verschmutzung bleibt.

Bei der Lösung dieser Aufgabe wird ausgegangen von einem Inhalationsgerät der eingangs erwähnten Art; gelöst wird die Aufgabe gemäß dem kennzeichnenden Teil des ersten Patentanspruchs durch das Vorsehen zumindest eines Luftauslaßkanals im oberen Abschnitt der Vorderseite des Gehäuses, dessen Austrittsspalt schräg nach unten weist, und wenigstens eines Luftauslaßschlitzes in dem an die Vorderseite angrenzenden Abschnitt des Gehäusebodens.

Luftauslaßkanal und Luftauslaßschlitz bilden zusammen die Luftaustrittsöffnungen für die das Gehäuseinnere durchströmende Kühlluft. Der durch die Lufteinlaßöffnungen im Bereich der Geräterückseite eingesogene Luftstrom teilt sich im Inneren des Gehäuses auf und strömt sowohl an der Ober- wie auch der Unterseite des Drucklufterzeugers bzw. des Elektromotors entlang. Aus dem oberen Bereich des Gehäuseinneren wird die aufgeiwärmte Kühlluft durch den im oberen Abschnitt der Vorderseite des Gehäuses vorgesehenen Luftauslaßkanal schräg nach unten und schließlich ins Freie abgeführt, während der übrige Teil der Kühlluft das Gehäuseinnere durch den Luftauslaßschlitz verläßt, welcher im vorderen Abschnitt des Gehäusebodens eingearbeitet ist. Dadurch, daß der Austrittsspalt des Luftauslaßkanals an der Vorderseite des Gehäuses schräg nach unten weist und wenigstens ein weiterer Luftauslaßschlitz im Gehäuseboden angeordnet ist, strömt die Kühlluft nicht, wie bei herkömmlichen Inhalationsgeräten, ungefähr waagrecht aus der dem Benutzer zugewandten Frontseite aus, sondern wird nach unten ausgelassen.

Durch diese besondere Führung des Kühlluftstroms ergeben sich mehrere Vorteile: Der vom Drucklufterzeuger und dem Elektromotor abgestrahlte Schall kann nicht mehr ungehindert nach vorne aus dem Gehäuse austreten, sondern wird im nach unten weisenden Luftauslaßkanal umgelenkt bzw. kann nur durch die Luftauslaßschlitze im Gehäuseboden nach außen dringen. Da die Betriebsgeräusche von Drucklufterzeuger und Elektromtor überwiegend hochfrequente Anteile aufweisen, werden diese Störgeräusche an den Innenwänden des Luftauslaßkanals sowie im Bereich der Luftauslaßschlitze mehrfach reflektiert, wodurch sich die gewünschte, beträchtliche Absenkung des Geräuschpegels ergibt. Erfindungsgemäß ausgebildete Inhalationsgeräte zeichnen sich deshalb durch eine um mindestens 5 Dezibel (A) verminderte Geräuschemission aus. Der Kühlluftstrom selbst wird jedoch nur unwesentlich behindert, so daß die mit der Durchströmung des Gehäuseinneren von der Rückseite zur Vorderseite hin grundsätzlich einhergehenden Vorteile - insbesondere Eindringen von ausgeatmeten Medikamentenpartikeln - erhalten bleiben. Die Aufteilung des Kühlluftstroms in einen entlang des Gehäusebodens streichenden unteren Teilstrom und einen innen entlang der Gehäuseoberseite geführten oberen Teilstrom gewährleistet eine optimale Abführung der vom Drucklufterzeuger bzw. dem Elektromotor im Betrieb erzeugten Verlustwärme. Das ausschließliche Ausblasen der Kühlluft schräg nach unten in Richtung des Gehäusebodens unterbindet einen direkten Luftzug in Richtung der vor dem Gerät sitzenden, inhalierenden Person. Die Gefahr des Einatmens von in der Kühlluft enthaltenen Schmutzpartikeln und eine Störung des Wohlbefindens des Patienten während des Inhalierens sind so ausgeschlossen. Die Ableitung der Kühlluft schräg nach unten verhindert überdies weitgehend eine Ablagerung von Schmutz- und Staubpartikeln auf der Frontseite des Geräts, welche dem Patienten unmittelbar vor Augen ist. Die Anordnung der Luftauslaßschlitze im Gehäuseboden und die Ausbildung des Luftauslaßkanals mit einem schräg nach unten weisenden Austrittsspalt erlaubt schließlich eine Gestaltung der dem Benutzer zugewandten Frontseite, welche bei Betrachtung von vorne bzw. schräg von oben überhaupt nicht erkennen läßt, daß Luftaustrittsöffnungen für Kühlluft im Bereich der Frontseite vorgesehen sind. Hierdurch wird eine in ästhetischer Hinsicht äußerst ansprechende Gestaltung des Gerätegehäuses ermöglicht.

Insgesamt zeichnet sich das erfindungsgemäße Inhalationsgerät also durch eine hochwirksame Kühlung des Drucklufterzeugers und des diesen antreibenden Elektromotors, wesentlich verminderte Geräuschemission, geringe Verschmutzungsneigung sowohl des Geräteinneren wie auch seiner Außenseite und eine ansprechende optische Gestaltung aus.

In zweckmäßiger Ausgestaltung des Erfindungsgegenstands ist der Luftauslaßkanal unter einem Winkel von 40 bis 70, vorzugsweise ungefähr 60, gegenüber dem Gehäuseboden nach unten geneigt. Ein so bemessener Neigungswinkel für den Luftauslaßkanal im oberen Abschnitt der Gehäusev orderseite ist ein guter Kompromiß zwischen möglichst ungehinderter Kühlluftströmung und wirkungsvoller Geräuschdämmung durch Schallumlenkung bzw. mehrfache Reflexion zwischen den Kanalwänden.

Zweckmäßigerweise verläuft der Austrittsspalt des Luftauslaßkanals parallel zur Oberkante der Vorderseite des Gehäuses. Der innen im Gehäuse entlang dessen Oberseite streichende Teil der Kühlluft gelangt so auf dem kürzesten Weg ins Freie, so daß dem Ausströmen der Kühlluft ein nur geringer Widerstand entgegengesetzt wird. Im Interesse eines möglichst hohen Kühlluftdurchsatzes erstreckt sich vorteilhafterweise der Austrittsspalt über annähernd die gesamte Breite des Gehäuses. Beträgt die Länge des Luftauslaßkanals wenigstens das Zweifache der Breite des Austrittsspalts, so wird die zwischen den Kanalwänden strömende Kühlluft wirksam schräg nach unten geleitet, so daß der Patient auch in relativ nahem Abstand zum Gerät keine Zugluft erhält. Diese Dimensionierung des Luftauslaßkanals hat sich auch als zweckmäßig im Hinblick auf die gewünschte Verringerung der Geräuschemission erwiesen.

In vorteilhafter Weiterbildung des erfindungsgemäßen Inhalationsgeräts ist kurz hinter und parallel zu dem Luftauslaßschlitz im Gehäuseboden ein nach unten weisender Quersteg vorgesehen. Dieser Quersteg am Gehäuseboden hinter dem Luftauslaßschlitz verhindert das Rückströmen der ausgeblasenen Kühlluft außen am Gehäuseboden entlang in Richtung der Gehäuserückseite; vorzugsweise steht dieser Quersteg rechtwinklig vom Gehäuseboden ab.

Weist der Luftauslaßschlitz schräg nach vorne in Richtung der Frontseite, so unterstützt dies ein möglichst ungehindertes Ausströmen der Kühlluft. Eine Neigung des Luftauslaßschlitzes um einen Winkel von ungefähr 45 gegenüber dem Gehäuseboden hat sich als optimal erwiesen.

Aus Stabilitätsgründen kann der Luftauslaßschlitz durch Zwischenstege unterteilt sein. Um einen möglichst großen Austrittsquerschnitt zu erreichen, erstreckt sich der Luftauslaßschlitz zweckmäßigerweise über annähernd die gesamte Breite des Gehäuses. Es können auch mehrere, beispielsweise zwei parallel nebeneinander angeordnete Luftauslaßschlitze vorgesehen sein.

Bei einer bevorzugten Ausführungsform des Inhalationsgeräts gemäß der Erfindung ist das Gehäuse aus einem Gehäuseunterteil, einem Gehäuseoberteil und einer Frontplatte zusammengesetzt, weist die Frontplatte im Bereich ihrer Oberkante einen zum Innern des Gehäuses hin abgewinkelten Frontplattenabschnitt auf, hat das Gehäuseoberteil einen bis in die Frontseite herabgezogenen Vorderabschnitt, der den abgewinkelten Frontplattenabschnitt überdeckt, und bildet der Vorderabschnitt des Gehäuseoberteils und der Frontplattenabschnitt gemeinsam den Luftauslaßkanal. Ein derartig ausgebildetes, aus nur drei Teilen zusammengesetztes Gehäuse läßt sich fertigungstechnisch leicht herstellen und auf einfachste Weise, nämlich durch Einstecken der Frontplatte und Aufsetzen des Gehäuseoberteils auf das Gehäuseunterteil, montieren. Der Luftauslaßkanal im oberen Abschnitt der Vorderseite der Gehäuses muß nicht in das Gehäuseoberteil bzw. in den oberen Teil der Frontplatte eingearbeitet werden, sondern ergibt sich von selbst aufgrund des Zusammenwirkens der beiden in gleichem Sinne abgewinkelten Abschnitte der Frontplatte bzw. des Gehäuseoberteils. Der quer über die Gehäusevorderseite verlaufende Austrittsspalt des Luftauslaßkanals zwischen Frontplatte und über diese herabgezogenem Gehäuseoberteil ist, von vorne bzw. schräg oben betrachtet, vollkommen verdeckt, so daß optisch der Eindruck einer vollständig geschlossenen Gehäusevorderfront entsteht. Die unteren Luftauslaßschlitze sind ohnehin von vorne nicht zu erkennen, da sie sich ja im Gehäuseboden befinden.

Die Montage des Gehäuses wird noch einfacher, wenn die Frontplatte zwischen das Gehäuseunterteil und das Gehäuseoberteil eingesteckt ist. Hierzu kann die Frontplatte an ihrer Oberkante schmale Distanzstege aufweisen, die in entsprechenden Nuten am Gehäuseoberteil eingreifen.

Die Lufteinlaßöffnungen, durch die kalte Umgebungsluft in das Gehäuseinnere strömt, umfassen zweckmäßigerweise Lufteinlaßschlitze, welche in die Rückseite des Gehäuses eingearbeitet sind. Um den Kühlluftstrom auf die zu kühlenden Aggregate, nämlich den Druckerzeuger und den diesen antreibenden Elektromotor zu konzentrieren, sind diese Lufteinlaßschlitze bevorzugt im mittleren Abschnitt der Rückseite angeordnet.

Ein Ausführungsbeispiel der Erfindung wird nachstehend anhand der beigefügten Zeichnungen näher erläutert. Es zeigen:

Fig. 1    ein Inhalationsgerät, in einer perspektivischen Ansicht;

Fig. 2    das Inhalationsgerät von Fig. 1, in einem Vertikalschnitt entlang der Linie II-II; und

Fig. 3    das dreiteilige Gehäuse des Inhalationsgeräts von Fig. 1, in einer explosionsartigen perspektivischen Darstellung.

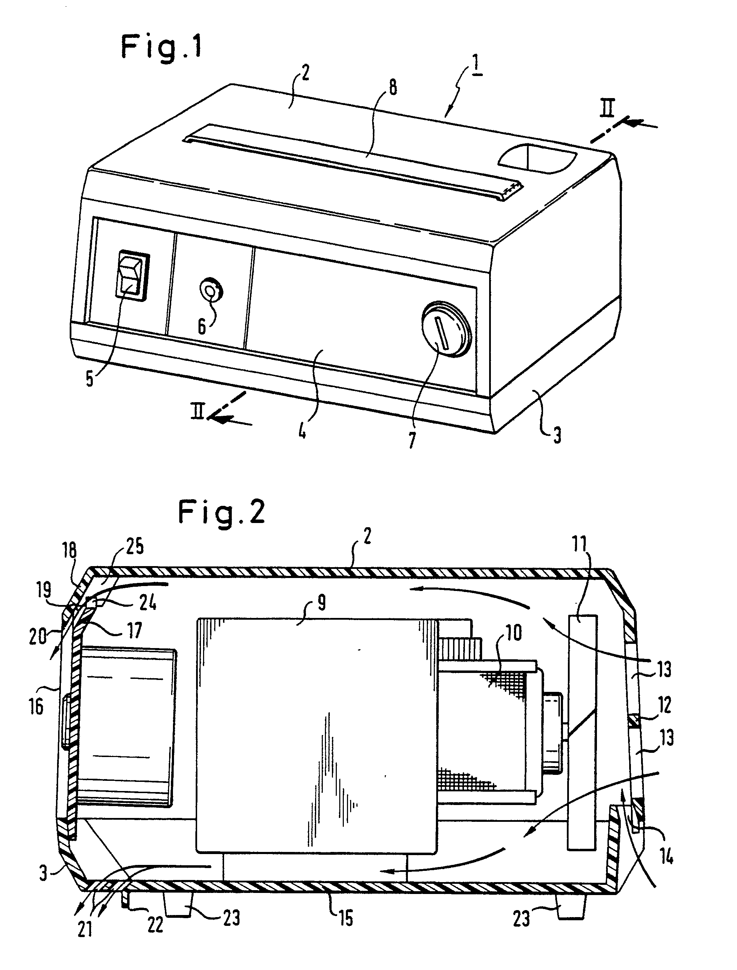

Das in Fig. 1 dargestellte Inhalationsgerät besitzt ein Gehäuse 1 in der Grundform eines Quaders. Das Gehäuse 1 st zusammengesetzt aus einem Gehäuseoberteil 2, einem Gehäuseunterteil 3 und einer zwischen diese eingesteckten Frontplatte 4. Auf der Frontplatte 4 ist ein Netzschalter 5, ein Druckluftanschluß 6 und ein austauschbares Luftfiter 7 angeordnet. Auf dem Gehäuseoberteil 2 ist ein Tragegriff 8 vorgesehen.

Fig. 2 zeigt einen Querschnitt durch das Inhalationsgerät. Das Gehäuse 1 umschließt einen Drucklufterzeuger 9, der hier als Membrankompressor ausgeführt ist und von einem Elektromotor 10 angetrieben wird. Ein Lüfterrad 11 sitzt stirnseitig auf der Welle des Elektromotors 10.

An der Rückseite 12 des Gehäuses 1 sind Lufteinlaßöffnungen vorgesehen. Diese Lufteinlaßöffnungen werden gebildet von zwei Reihen übereinander angeordneten, vertikalen Lufteinlaßschlitzen 13, welche in das Gehäuseoberteil 2 eingearbeitet sind, sowie einem weiteren Lufteinlaßschlitz 14, welcher zwischen dem Gehäuseoberteil 2 und dem in diesem Bereich zurückspringenden Gehäuseunterteil 2 vorgesehen ist. Nach Inbetriebnahme des Inhalationsgeräts saugt das vom Elektromotor 10 angetriebene Lüfterrad 11 Kühlluft durch die Lufteinlaßschlitze 13, 14 auf der Rückseite 12 des Gehäuses 1 an; der Kühlluftstrom teilt sich auf in einen ersten Teilstrom, der entlang der Innenseite des Gehäuseoberteils 2 am Drucklufterzeuger 9 vorbei strömt, und einem zweiten Teilstrom, welcher entlang des Gehäusebodens 15 in Richtung der Frontseite 16 streicht. Die Frontplatte 4 besitzt einen Frontplattenabschnitt 17, der im Bereich ihrer Oberkante zum Innern des Gehäuses 1 hin abgewinkelt ist. Das Gehäuseoberteil 2 ist bis in die Frontseite 16 herabgezogen und überdeckt mit einem entsprechend abgewinkelten Vorderabschnitt 18 den Frontplattenabschnitt 17. Vorderabschnitt 18 des Gehäuseoberteils 2 und Frontplattenabschnitt 17 bilden gemeinsam einen Luftauslaßkanal 19, dessen Austrittsspalt 20 unter einem Winkel von ungefähr 60 gegenüber dem Gehäuseboden 15 schräg nach unten weist. In dem an die Frontseite 16 angrenzenden Abschnitt des Gehäusebodens 15 sind zwei parallele Luftauslaßschlitze 21 vorgesehen, welche sich über die gesamte Breite des Gehäuses 1 erstrecken. Diese Luftauslaßschlitze 21 sind um einen Winkel von ungefähr 45 gegenüber dem Gehäuseboden 15 schräg nach vorne in Richtung der Frontseite 16 geneigt. Die gesamte, durch die Lufteinlaßschlitze 13, 14 in das Innere des Gehäuses 1 gesaugte Kühlluft wird durch den Luftauslaßkanal 19 zwischen Gehäuseoberteil 2 und Frontplatte 4 sowie die Luftauslaßschlitze 21 im Gehäuseunterteil 3 schräg nach vorne unten ausgeblasen. Kurz hinter den Luftauslaßschlitzen 21 im Gehäuseunterteil 3 ist ein Quersteg 22 vorgesehen, der rechtwinklig vom Gehäuseboden 15 absteht. Dieser Quersteg 22 verhindert ein Rückströmen von durch die Luftauslaßschlitze 21 ausgeblasener Kühlluft in Richtung der Rückseite 12 des Gehäuses 1. Im Geräteinneren aufgeheizte Kühlluft kann so nicht noch einmal über die Lufteinlaßschlitze 13, 14 in das Innere des Gehäuses 1 gela ngen. Am Gehäuseboden 15 sind Füße 23 vorgesehen, mit denen das Inhalationsgerät auf seiner jeweiligen Stellfläche so aufsteht, daß die Unterseite frei ist.

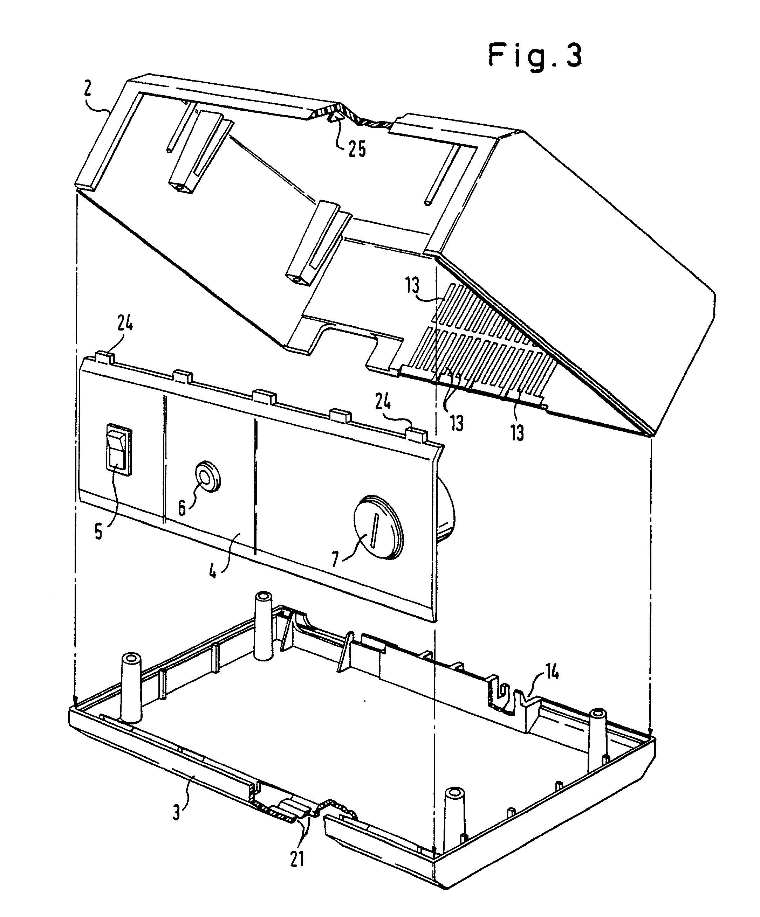

Fig. 3 verdeutlicht die leichte und schnelle Montage des Gehäuses 1 des Inhalationsgeräts. Das Gehäuse 1 ist lediglich aus drei Teilen, nämlich dem Gehäuseoberteil 2, dem Gehäuseunterteil 3 und der Frontplatte 4 zusammengesetzt. Mit ihrer Unterkante wird die Frontplatte 4 einfach in das Gehäuseunterteil 3 eingesteckt. An der Oberkante ihres nach innen abgewinkelten Frontplattenabschnitts 17 weist die Frontplatte 4 schmale Distanzstege 24 auf; in zusammenmontiertem Zustand greifen diese Distanzstege 24 in entsprechende Nuten 25 ein, welche an der vorderen Oberkante des Gehäuseoberteils 2 ausgebildet sind. Das Gehäuseoberteil 2 ist seitlich so tief herabgezogen, daß es die Frontplatte 4 an drei Seiten rahmenartig umgibt. Die Lufteinlaßschlitze 13 sind im mittleren Abschnitt der Rückseite 12 eingearbeitet; der zusätzliche Lufteinlaßschlitz 14 ist zwischen dem Gehäuseoberteil 2 und dem Gehäuseunterteil 3, ebenfalls im mittleren Abschnitt der Rückseite 12, angeordnet.

## Ansprüche

1. Inhalationsgerät, mit
   - einem Drucklufterzeuger (9),
   - einem Elektromotor (10), der den Drucklufterzeuger (9) antreibt,
   - einem im wesentlichen quaderförmigen Gehäuse (1), das im Bereich seiner Rückseite (12) Lufteinlaßöffnungen und im Bereich seiner dem Benutzer zugewandten Frontseite (16) Luftaustrittsöffnungen aufweist, so daß Kühlluft im wesentlichen horizontal von der Rückseite (12) zur Frontseite (16) hin durch das Innere des Gehäuses (1) strömt, **gekennzeichnet** durch
   - zumindest einen Luftauslaßkanal (19) im oberen Abschnitt der Frontseite (16) des Gehäuses (1), dessen Austrittsspalt (20) schräg nach unten weist, und
   - wenigstens einen Luftauslaßschlitz (21), in dem an die Frontseite (16) angrenzenden Abschnitt des Gehäusebodens (15).

2. Inhalationsgerät nach Anspruch 1, dadurch gekennzeichnet, daß der Luftauslaßkanal (19) unter einem Winkel von 40 bis 70 gegenüber dem Gehäuseboden (15) nach unten geneigt ist.

3. Inhalatationsgerät nach Anspruch 2, dadurch gekennzeichnet, daß der Luftauslaßkanal (19) unter einem Winkel von ungefähr 60 gegenüber dem Gehäuseboden (15) nach unten geneigt ist.

4. Inhalationsgerät nach einem der Ansprüche 1 bis 3, dadurch gekennzeichnet, daß der Austrittsspalt (20) des Luftauslaßkanals (19) parallel zur Oberkante der Frontseite (16) des Gehäuses (1) verläuft.

5. Inhalationsgerät nach Anspruch 4, dadurch gekennzeichnet, daß sich der Austrittsspalt (20) über annähernd die gesamte Breite des Gehäuses (1) erstreckt.

6. Inhalationsgerät nach einem der Ansprüche 1 bis 5, dadurch gekennzeichnet, daß die Länge des Luftauslaßkanals (19) wenigstens das Zweifache der Breite des Austrittsspalts (20) beträgt.

7. Inhalationsgerät nach einem der Ansprüche 1 bis 6, dadurch gekennzeichnet, daß kurz hinter und parallel zu dem Luftauslaßschlitz (21) im Gehäuseboden (15) ein nach unten weisender Quersteg (22) vorgesehen ist.

8. Inhalationsgerät nach Anspruch 7, dadurch gekennzeichnet, daß der Quersteg (22) rechtwinklig vom Gehäuseboden (15) absteht.

9. Inhalatationsgerät nach einem der Ansprüche 1 bis 8, dadurch gekennzeichnet, daß der Luftauslaßschlitz (21) schräg nach vorne in Richtung der Frontseite (16) weist.

10. Inhalationsgerät nach Anspruch 9, dadurch gekennzeichnet, daß der Luftauslaßschlitz (21) um einen Winkel von ungefähr 45 gegenüber dem Gehäuseboden (15) geneigt ist.

11. Inhalationsgerät nach einem der Ansprüche 1 bis 10, dadurch gekennzeichnet, daß der Luftauslaßschlitz (21) durch Zwischenstege unterteilt ist.

12. Inhalationsgerät nach einem der Ansprüche 1 bis 11, dadurch gekennzeichnet, daß sich der Luftauslaßschlitz (21) über annähernd die gesamte Breite des Gehäuses (1) erstreckt.

13. Inhalationsgerät nach einem der Ansprüche 1 bis 12, dadurch gekennzeichnet, daß mehrere, parallel nebeneinander angeordnete Luftauslaßschlitze (21) vorgesehen sind.

14. Inhalationsgerät nach einem der Ansprüche 1 bis 13, dadurch gekennzeichnet, daß
- das Gehäuse (1) aus einem Gehäuseoberteil (2), einem Gehäuseunterteil (3) und einer Frontplatte (4) zusammengesetzt ist,
- die Frontplatte (4) im Bereich ihrer Oberkante einen zum Inneren des Gehäuses (1) hin abgewinkelten Frontplattenabschnitt (17) aufweist,
- das Gehäuseoberteil (2) einen bis in die Frontseite (16) herabgezogenen Vorderabschnitt (18) aufweist, der den abgewinkelten Frontplattenabschnitt (17) überdeckt, und
- der Vorderabschnitt (18) des Gehäuseoberteils (2) und der Frontplattenabschnitt (17) gemeinsam den Luftauslaßkanal (19) bilden.

15. Inhalationsgerät nach Anspruch 14, dadurch gekennzeichnet, daß die Frontplatte (4) zwischen das Gehäuseunterteil (3) und das Gehäuseoberteil (2) eingesteckt ist.

16. Inhalationsgerät nach Anspruch 15, dadurch gekennzeichnet, daß die Frontplatte (4) an ihrer Oberkante schmale Distanzstege (24) aufweist, die in entsprechende Nuten (25) am Gehäuseoberteil (2) eingreifen.

17. Inhalationsgerät nach einem der Ansprüche 1 bis 16, dadurch gekennzeichnet, daß die Lufteinlaßöffnungen in die Rückseite (12) des Gehäuses (1) eingearbeitete Lufteinlaßschlitze (13, 14) umfassen.

18. Inhalationsgerät nach Anspruch 17, dadurch gekennzeichnet, daß die Lufteinlaßschlitze (13, 14) im mittleren Abschnitt der Rückseite (12) angeordnet sind.

## Claims

1. Inhaler, with
- an air compressor (9),
- an electric motor (10) which drives the air compressor (9),
- an essentially cuboid housing (1) which comprises air inlet openings in the region of its rear side (12) and air outlet openings in the region of its front side (16) facing towards the user, so that cooling air flows essentially horizontally from the rear side (12) to the front side (16) through the interior of the housing (1), characterised by
- at least one air outlet channel (19) in the upper section of the front side (16) of the housing (1), the outlet aperture (20) of which points obliquely downwards, and
- at least one air outlet slot (21), in the section of the bottom (15) of the housing adjoining the front side (16).

2. Inhaler according to claim 1, characterised in that the air outlet channel (19) is inclined downwards at an angle of 40 to 70° to the bottom (15) of the housing.

3. Inhaler according to claim 2, characterised in that the air outlet channel (19) is inclined downwards at an angle of about 60° to the bottom (15) of the housing.

4. Inhaler according to any of claims 1 to 3, characterised in that the outlet aperture (20) of the air outlet channel (19) extends parallel to the upper edge of the front side (16) of the housing (1).

5. Inhaler according to claim 4, characterised in that the outlet aperture (20) extends across almost the full width of the housing (1).

6. Inhaler according to any of claims 1 to 5, characterised in that the length of the air outlet channel (19) is at least twice the width of the outlet aperture (20).

7. Inhaler according to any of claims 1 to 6, characterised in that a downwardly pointing transverse web (22) is provided just behind and parallel to the air outlet slot (21) in the bottom (15) of the housing.

8. Inhaler according to claim 7, characterised in that the transverse web (22) projects from the bottom (15) of the housing at a right angle.

9. Inhaler according to any of claims 1 to 8, characterised in that the air outlet slot (21) points obliquely forwards in the direction of the front side (16).

10. Inhaler according to claim 9, characterised in that the air outlet slot (21) is inclined by an angle of about 450 to the bottom (15) of the housing.

11. Inhaler according to any of claims 1 to 10, characterised in that the air outlet slot (21) is divided by intermediate webs.

12. Inhaler according to any of claims 1 to 11, characterised in that the air outlet slot (21) extends across almost the full width of the housing (1).

13. Inhaler according to any of claims 1 to 12, characterised in that more than one air outlet slot (21) is provided, parallel and adjacent to each other.

14. Inhaler according to any of claims 1 to 13, characterised in that - the housing (1) is composed of an upper portion (21), a lower portion (3) and a front panel (4),
   - the front panel (4) comprises in the region of its upper edge a front panel section (17) bent at an angle towards the interior of the housing (1),
   - the upper portion (2) of the housing has a front section (18) which extends downwards as far as the front side (16) and which overlaps the angled front panel section (17), and
   - the front section (18) of the housing upper portion (2) and the front panel section (17) together form the air outlet channel (19).

15. Inhaler according to claim 14, characterised in that the front panel (4) is inserted between the lower (3) and upper (2) portions of the housing.

16. Inhaler according to claim 15, characterised in that the front panel (4) comprises, at its upper edge, narrow spacer webs (24) which engage in corresponding grooves (25) in the upper portion (2) of the housing.

17. Inhaler according to any of claims 1 to 16, characterised in that the air inlet openings include air inlet slots (13, 14) which are formed in the rear side (12) of the housing (1).

18. Inhaler according to claim 17, characterised in that the air inlet slots (13, 14) are arranged in the central section of the rear side (12).


**Revendications**

1. Appareil d'inhalation, comportant
   - un générateur d'air comprimé (9),
   - un moteur électrique (10) qui entraîne 1e générateur d'air comprimé (9),
   - un carter (1) sensiblement en forme de parallélépipède rectangle qui présente, dans la zone de sa face arrière (12) des ouvertures d'admission d'air et dans la zone de sa face avant (16), tournée vers l'utilisateur, des ouvertures de sortie d'air, de sorte que l'air de refroidissement s'écoule sensiblement horizontalement, de la face arrière (12) à la face avant (16), en passant par l'intérieur du carter (1), caractérisé par
   - au moins un canal de sortie d'air (19) situé dans la section supérieure de la face avant (16) du carter (1), dont l'interstice de sortie (20) est incliné vers le bas,
   - au moins une fente de sortie d'air (21) dans la section du fond de carter (15) limitrophe de la face avant (16).

2. Appareil d'inhalation selon la revendication 1, caractérisé en ce que le canal de sortie d'air (19) est incliné vers le bas, en faisant un angle de 40 à 70° par rapport au fond du carter (15).

3. Appareil d'inhalation selon la revendication 2, caractérisé en ce que le canal de sortie d'air (19) est incliné vers le bas en faisant un angle d'à peu près 60° par rapport au fond du carter (15).

4. Appareil d'inhalation selon l'une des revendications 1 à 3, caractérisé en ce que l'interstice de sortie d'air (20) du canal de sortie d'air (19) s'étend parallèlement à l'arête supérieure de la face avant (16) du carter (1).

5. Appareil d'inhalation selon la revendication 4, caractérisé en ce que l'interstice de sortie (20) s'étend sur à peu près la totalité de la largeur du carter (1).

6. Appareil d'inhalation selon l'une des revendications 1 à 5, caractérisé en ce que la longueur du canal de sortie d'air (19) est au moins du double de la largeur de l'interstice de sortie (20).

7. Appareil d'inhalation selon l'une des revendications 1 à 6, caractérisé en ce que peu en arrière et parallèlement à la fente de sortie d'air (21) est prévue dans le fond de carter (15) une nervure transversale (22) tournée vers le bas.

8. Appareil d'inhalation selon la revendication 7, caractérisé en ce que la nervure transversale (22) s'écarte à angle droit du fond de carter (15).

9. Appareil d'inhalation selon l'une des revendications 1 à 8, caractérisé en ce que la fente de sortie d'air (21) est inclinée vers l'avant en direction de la face avant (16).

10. Appareil d'inhalation selon la revendication 9, caractérisé en ce que la fente de sortie d'air (21) est inclinée par rapport au fond de carter (15) d'un angle d'à peu près 45°.

11. Appareil d'inhalation selon l'une des revendications 1 à 10, caractérisé en ce que la fente de sortie d'air (21) est subdivisée par des nervures intermédiaires.

12. Appareil d'inhalation selon l'une des revendications 1 à 11, caractérisé en ce que la fente de sortie d'air (21) s'étend sur à peu près la totalité de la largeur du carter (1).

13. Appareil d'inhalation selon l'une des revendications 1 à 12, caractérisé en ce que plusieurs fentes de sortie d'air (21) disposées parallèlement les unes à côté des autres sont prévues.

14. Appareil d'inhalation selon l'une des revendications 1 à 13, caractérisé en ce que
   - le carter (1) se compose d'une partie supérieure de carter (2), d'une partie inférieure de carter (3) et d'une plaque avant (4),
   - dans la zone de son arête supérieure, la plaque avant (4) présente une section de plaque avant (17) repliée vers l'intérieur du carter (1),
   - la partie supérieure de carter (2) présente une section avant (18), qui descend jusqu'à la face avant (16) et recouvre la section de plaque avant (17) repliée, et
   - la section avant (18) de la partie supérieure de carter (2) et la section de plaque avant (17) forment en commun le canal de sortie d'air (19).

15. Appareil d'inhalation selon la revendication 14, caractérisé en ce que la plaque avant (4) est enfichée entre la partie inférieure de carter (3) et la partie supérieure de carter (2).

16. Appareil d'inhalation selon la revendication 15, caractérisé en ce que, sur son arête supérieure, la plaque avant (4) présente d'étroites nervures d'espacement (24) qui s'engagent dans des gorges (25) correspondantes situées sur la partie supérieure de carter (2).

17. Appareil d'inhalation selon l'une des revendications 1 à 16, caractérisé en ce que les ouvertures d'admission d'air comprennent des fentes d'admission d'air (13,14) usinées dans la face arrière (12) du carter (1).

18. Appareil d'inhalation selon la revendication 17, caractérisé en ce que les ouvertures d'admission d'air (13,14) sont disposées dans la section centrale de la face arrière (12).

# Fig.1

# Fig.2

# Fig. 3